# EUROPEAN PATENT APPLICATION

(11) **EP 4 397 257 A1**
(43) Date of publication of application: **10.07.2024**
(21) Application number: 22863221.2
(22) Date of filing: 23.08.2022
(51) Int. Cl.: A61B 17/3203

(54) **MEDICAL WATER JET AND MEDICAL SYSTEM**

(30) Priority: 31.08.2021 CN 202111008949
(71) Applicant: Bluesail Surgical Co., Ltd., Shanghai 201321 (CN)
(72) Inventor: DING, Weijiang, Shanghai 201321 (CN); YANG, Shenghua, Shanghai 201321 (CN)
(74) Representative: Viering, Jentschura & Partner mbB Patent- und Rechtsanwälte
(86) International application number: PCT/CN2022/114121
(87) International publication number: WO 2023/030082

(57) **Abstract**

A medical water jet (100) and a medical system. The medical water jet (100) comprises a nozzle (110) and a jet pipe (120), wherein the nozzle (110) comprises a plurality of jet holes (111); and the jet pipe (120) comprises an input end (121a), an output end, and an inner cavity (121) configured to provide a space for a medical liquid to pass through. The input end (121a) of the jet pipe (120) is configured to allow the medical liquid to flow into same; and the output end of the jet pipe (120) is in communication with the plurality of jet holes (111), so that the medical liquid is jetted through the plurality of jet holes (111). The medical water jet (100) is capable of realizing multi-jet hole selective separation, which, compared with existing single-jet hole separation, makes flexible tissue to be separated not prone to escaping from an impact area of a multi-jet hole jet; therefore, the cutting of the flexible tissue and the separation of same from lumen-type elastic tissue are completed in a relatively stationary manner, thereby improving the tissue selective separation performance of the water jet and the surgical cutting efficiency of the medical water jet (100), and simplifying surgical operation. In addition, the problem of the water jet not being able to work after the jet holes (111) are blocked is also solved.

## Description

### CROSS-REFERENCE TO RELATED APPLICATION(S)

The present application claims priority to China patent application No.202111008949.2 filed on August 31, 2021, the disclosure of which is incorporated herein by reference in its entirety as part of the present application, for all purposes.

### TECHNICAL FIELD

Embodiments of the present disclosure relate to a medical water jet scalpel and a medical system.

### BACKGROUND

At present, cancer is one of the major diseases that have not been conquered by modern medicine. According to some cancer statistics, liver cancer ranks the fifth in incidence and the second in mortality among various cancer diseases. The high incidence and mortality of liver cancer reflect the fact that there is still absent of effective and targeted therapeutic methods currently.

Therapeutic methods for liver cancer include surgical treatment, local ablation, transcatheter arterial chemoembolization, radiation therapy, systemic therapy and the like, among which surgical treatment remains as a preferred one for liver cancer.

The key operating steps of a surgical treatment include dissociation of liver from ligaments, blocking of inflow of blood vessels and bile ducts (to the first hepatic portal), liver resection, blocking of outflow of blood vessels and bile ducts (to the second hepatic portal and the third hepatic portal), and abdominal closure after complete hemostasis. However, a surgery may also lead to complications such as infection, bleeding, bile leakage, and liver failure, which has seriously affected the five-year survival rate and the mortality rate of liver cancer. From the aspects of operation difficulty, time consumption and influence on complications of these key steps, liver resection is the most noteworthy.

At present, surgical treatments for liver resection are mainly divided into three categories, and there are more than ten specific methods. For example, the commonly used methods include: (1) separation of liver parenchyma and preservation of blood vessels; (2) separation of liver parenchyma, and closure and cutting of blood vessels and bile ducts; (3) coagulation of resected surface of liver parenchyma, and cutting of the same with a scalpel.

### SUMMARY

At least one embodiment of the present disclosure provides a medical water jet scalpel including a water jet nozzle and a water jet tube. The water jet tube includes an inlet, an outlet, and an inner lumen configured to provide space for circulation of medical liquid; the inlet of the water jet tube is configured for an inflow of the medical liquid; the water jet nozzle includes a plurality of orifices, the plurality of orifices are communicated with the outlet of the water jet tube, and the plurality of orifices are configured to eject the medical liquid passing through the outlet of the water jet tube.

For example, in the medical water jet scalpel provided by at least one embodiment of the present disclosure, the water jet nozzle includes a multi-orifice sub-nozzle provided with the plurality of orifices.

For example, in the medical water jet scalpel provided by at least one embodiment of the present disclosure, the water jet nozzle includes a plurality of single-orifice sub-nozzles, and at least part of the plurality of orifices are respectively formed in the plurality of single-orifice sub-nozzles.

For example, in the medical water jet scalpel provided by at least one embodiment of the present disclosure, the outlet of the water jet tube is a blind end, and at least part of the blind end serves as the water jet nozzle.

For example, in the medical water jet scalpel provided by at least one embodiment of the present disclosure, the outlet of the water jet tube is a blind end, and the water jet nozzle is arranged at the blind end of the water jet tube.

For example, in the medical water jet scalpel provided by at least one embodiment of the present disclosure, the water jet tube is a sleeved tube opened at both ends, the water jet nozzle is fixed at the outlet of the water jet tube, and the water jet nozzle is connected with the outlet of the water jet tube in a sealed manner, directly or indirectly.

For example, the medical water jet scalpel provided by at least one embodiment of the present disclosure further includes an outer aspiration tube, wherein the outer aspiration tube is sleeved outside the water jet tube, and a gap space between an outer sidewall of the water jet tube and an inner sidewall of the outer aspiration tube serves as an aspiration channel.

For example, in the medical water jet scalpel provided by at least one embodiment of the present disclosure, an inner diameter of the outer aspiration tube is 3 mm to 7.6 mm, and an outer diameter of the outer aspiration tube is 4 mm to 8 mm; an inner diameter of the water jet tube is 1.5 mm to 5 mm, and an outer diameter of the water jet tube is 2 mm to 6 mm; and a diameter of the orifice is 0.05 mm to 0.15 mm.

For example, in the medical water jet scalpel provided by at least one embodiment of the present disclosure, the plurality of orifices are uniformly arranged.

For example, in the medical water jet scalpel provided by at least one embodiment of the present disclosure, a distance between centers of two adjacent orifices among the plurality of orifices is 0.4 mm to 4.0 mm.

For example, in the medical water jet scalpel provided by at least one embodiment of the present disclosure, a flow rate of jet of the medical liquid ejected from the plurality of orifices is 50 m/s to 100 m/s.

For example, in the medical water jet scalpel provided by at least one embodiment of the present disclosure, the water jet nozzle includes one or more of the following materials: metal, gemstone and plastic.

For example, in the medical water jet scalpel provided by at least one embodiment of the present disclosure, the water jet tube includes one or more of the following materials: metal and plastic.

For example, the medical water jet scalpel provided by at least one embodiment of the present disclosure further includes a nested joint, wherein the nested joint is fixedly connected with the water jet tube in a sealed manner, and the water jet nozzle is connected with the water jet tube through the nested j oint; an output end of the nested joint is provided with a counterbore, the water jet nozzle is arranged in the counterbore of the nested joint, and a sidewall of the counterbore wraps the water jet nozzle; and the plurality of orifices, the nested joint and the water jet tube are sequentially communicated along multiple axes of the plurality of orifices parallel to the water jet tube.

For example, in the medical water jet scalpel provided by at least one embodiment of the present disclosure, the outlet of the water jet tube is in interference fit with the water jet nozzle.

For example, in the medical water jet scalpel provided by at least one embodiment of the present disclosure, the water jet nozzle is fixed at an inner side of the outlet of the water jet tube in a sealed manner.

For example, the medical water jet scalpel provided by at least one embodiment of the present disclosure further includes a protective cover which is opened at both ends, wherein the outlet of the water jet tube is provided with a first counterbore, and the protective cover is embedded in the first counterbore of the water jet tube; an output end of the protective cover is provided with a second counterbore, and the water jet nozzle is embedded in the second counterbore of the protective cover; and the plurality of orifices, the protective cover and the water jet tube are sequentially communicated along multiple axes of the plurality of orifices parallel to the water jet tube.

For example, in the medical water jet scalpel provided by at least one embodiment of the present disclosure, the blind end of the water jet tube includes one blind end surface and a plurality of stepped holes, wherein the stepped hole includes a first subspace protruded from the blind end surface and a second subspace recessed into the blind end surface; and each single-orifice sub-nozzle of the plurality of single-orifice sub-nozzles included in the water jet nozzle is embedded in the first subspace of a corresponding stepped hole of the plurality of stepped holes.

For example, the medical water jet scalpel provided by at least one embodiment of the present disclosure further includes a support sleeve, wherein the outlet of the water jet tube is configured as a step structure, the support sleeve is matched with the step structure of the water jet tube in shape and protruded into the inner lumen of the water jet tube, and the water jet nozzle is fixedly connected at an output end of the support sleeve; and a part of a space outside an integral structure constituted by connecting the step structure, the support sleeve and the water jet nozzle is used as an injection molding region.

For example, the medical water jet scalpel provided by at least one embodiment of the present disclosure further includes a handle, wherein the side of the outer aspiration tube away from the water jet nozzle is fixedly connected with the handle.

For example, the medical water jet scalpel provided by at least one embodiment of the present disclosure further includes an aspiration connecting tube, wherein the aspiration connecting tube is communicated with an end of the outer aspiration tube away from the water jet nozzle.

For example, the medical water jet scalpel provided by at least one embodiment of the present disclosure further includes an ejection connecting tube, wherein the ejection connecting tube is communicated with an end of the water jet tube away from the water jet nozzle.

For example, in the medical water jet scalpel provided by at least one embodiment of the present disclosure, the plurality of orifices include at least two orifices configured to eject the medical liquid to selectively separate the target tissue.

For example, at least one embodiment of the present disclosure further provides a medical system, including: the medical water jet scalpel described in any of the above; a liquid supply unit configured to provide the medical liquid; and a pressure pump configured to apply a pressure to the medical liquid to input the medical liquid into the inner lumen of the water jet tube.

For example, the medical system provided by at least one embodiment of the present disclosure further includes a wastewater reservoir, wherein the medical water jet scalpel also includes an outer aspiration tube, the outer aspiration tube is sleeved outside the water jet tube, and a gap space between an outer sidewall of the water jet tube and an inner sidewall of the outer aspiration tube is used as an aspiration channel, and the wastewater reservoir is communicated with the outer aspiration tube.

For example, the medical system provided by at least one embodiment of the present disclosure further includes a negative pressure aspirator, wherein the negative pressure aspirator is connected to the wastewater reservoir.

Compared with the prior art, at least one embodiment of the present disclosure has the following beneficial effects: the medical water jet scalpel according to the present disclosure can realize multi-orifice cutting, soft tissues to be cut are not easy to escape from an impact area of the multi-orifice jet, so that the cutting of the soft tissues and the separation of the soft tissues from lumen-like elastic tissues can be completed in a relatively fixed manner, the performance of the water jet scalpel for selective tissue separation is enhanced, the surgical efficiency of medical water jet cutting is improved, the surgical operation is simplified, and the problem that the water jet scalpel cannot work in case of orifice blockage can be solved.

### BRIEF DESCRIPTION OF DRAWINGS

In order to more clearly illustrate the technical solutions of the embodiments of the present disclosure or the prior art, the drawings necessary for description of the embodiments or the prior art will be briefly described in the following; it is obvious that the described drawings as below are only related to some embodiments of the present disclosure, from which other drawings can be obtained for those ordinary skilled in the art without creative labors.
Fig. 1 is a schematic diagram illustrating constituted components of a medical system provided by some embodiments of the present disclosure;
Fig. 2 is a schematic structural diagram of a medical water jet scalpel provided by some embodiments of the present disclosure;
Fig. 3a is a schematic diagram of a first type of structure of a water jet nozzle and a water jet tube provided by some embodiments of the present disclosure, and Fig. 3b is a left side view of the first type of structure as shown in Fig. 3a;
Fig. 4a is a schematic diagram illustrating a uniform arrangement of three orifices provided by some embodiments of the present disclosure;
Fig. 4b is a schematic diagram illustrating a uniform arrangement of four orifices provided by some embodiments of the present disclosure;
Fig. 4c is a schematic diagram illustrating a uniform arrangement of five orifices provided by some embodiments of the present disclosure;
Fig. 4d is a schematic diagram illustrating a uniform arrangement of seven orifices provided by some embodiments of the present disclosure;
Fig. 4e is a schematic diagram illustrating a uniform arrangement of nine orifices provided by some embodiments of the present disclosure;
Fig. 4f is a schematic diagram illustrating a uniform arrangement of twenty-one orifices provided by some embodiments of the present disclosure;
Fig. 5a is a schematic diagram of a second type of structure of a water jet nozzle and a water jet tube provided by some embodiments of the present disclosure, and Fig. 5b is a left side view of the second type of structure as shown in Fig. 5a;
Fig. 6a is a schematic diagram of a third type of structure of a water jet nozzle and a water jet tube provided by some embodiments of the present disclosure, and Fig. 6b is a left side view of the third type of structure as shown in Fig. 6a;
Fig. 7a is a schematic diagram illustrating a fourth type of structure of a water jet nozzle and a water jet tube provide by some embodiments of the present disclosure, and Figs. 7b and 7c are schematic diagrams illustrating sections A-A and B-B, respectively, of the fourth type of structure as shown in Fig. 7a;
Fig. 8a is a schematic diagram of a fifth type of structure of a water jet nozzle and a water jet tube provided by some embodiments of the present disclosure, and Fig. 8b is a left side view of the fifth type of structure as shown in Fig. 8a;
Fig. 9a is a schematic diagram of a sixth type of structure of a water jet nozzle and a water jet tube provided by some embodiments of the present disclosure, and Fig. 9b is a left side view of the sixth type of structure as shown in Fig. 9a;
Fig. 10a is a schematic diagram of a seventh type of structure of a water jet nozzle and a water jet tube provided by some embodiments of the present disclosure, and Fig. 10b is a left side view of the seventh type of structure as shown in Fig. 10a;
Fig. 11a is a schematic diagram of an eighth type of structure of a water jet nozzle and a water jet tube provided by some embodiments of the present disclosure, and Fig. 11b is a left side view of the eighth type of structure as shown in Fig. 11a;
Fig. 12 is a schematic table illustrating comparison results of separation efficiency and capability of a medical single-orifice water jet scalpel vs a medical multi-orifice water jet scalpel, as provided by some embodiments of the present disclosure; and
Fig. 13 is a schematic diagram illustrating an operation path of a medical single-orifice water jet scalpel and an operation path of a medical multi-orifice water jet scalpel, as provided by some embodiments of the present disclosure;
Fig. 14 is a schematic diagram illustrating processing effects of a medical multi-orifice water jet scalpel provided by some embodiments of the present disclosure; and
Fig. 15 is a schematic diagram illustrating processing effects of a medical single-orifice water jet scalpel provided by some embodiments of the present disclosure.

### DETAILED DESCRIPTION

Hereinafter, the technical solutions of the embodiments of the present disclosure will be described in a clearly and fully understandable way in connection with the drawings related to the embodiments of the present disclosure. Apparently, the described embodiments are just a part but not all of the embodiments of the present disclosure. Based on the described embodiments herein, all other embodiment(s) that can be obtained by those ordinary skilled in the art without any inventive work should be fallen within the scope of protection of the present disclosure.

Unless otherwise defined, all terms (including technical terms and scientific terms) used in the embodiments of the present disclosure have the same meanings as commonly understood by one of ordinary skilled in the art to which the present disclosure belongs. It should also be understood that terms such as those defined in a general dictionary should be interpreted to have meanings consistent with their meanings in the context of the relevant art, and are not to be interpreted in an idealized or overly formal sense, unless an embodiment of the present disclosure is explicitly defined so.

As used in the embodiments of the present disclosure, words such as "first", "second" and similarities do not indicate any order, quantity, or importance, but rather are used to distinguish between different components. Similar words such as "a", "an" or "the" do not indicate a limitation of quantity, but rather indicate the presence of at least one. Similarly, the use of the terms "comprising" or "comprise" and the like, is intended to mean that an element or article appearing before the word covers the listed element or article appearing after the word and its equivalents, but does not exclude other elements or articles. Similar terms such as "connect" or "connected" are not limited to physical or mechanical connections, but may include electrical or communicative connections, whether direct or indirect. Flowcharts are used in the embodiments of the present disclosure to illustrate steps of a method in accordance with the embodiments of the present disclosure. It should be understood that the preceding or following steps are not necessarily performed precisely in order. Rather, various steps may be carried out in reverse order or concurrently, unless the embodiments of the present disclosure are explicitly defined. Other operations can be added to these processes, or one or more steps are removed from these processes.

As used in the embodiments of the present disclosure, "inlet" refers to an end of each component or element or device or apparatus, for example, that is closer to a liquid inflow side, and "outlet" refers to an end of each component or element or device or apparatus, for example, that is away from the liquid inflow side. As used in the embodiments of the present disclosure, "blind end" refers to an end of each component or element or device or apparatus, for example, that is at least partly closed or enclosed, for example, the entirety of the end may be closed or enclosed, or a first portion of the end may be closed or enclosed while a second portion of the end may not be closed or enclosed.

With the development of the field of medical surgery towards the direction of minimal invasion and refinement, there is still no method that can fully satisfy the surgical requirements for liver resection, effectively control the postoperative complications and improve the five-year survival rate after surgery among numerous liver resection methods. Therefore, disruptive innovations in existing medical technologies are imperative under current circumstances.

Ideal methods of liver resection should have the following characteristics: high speed, no thermal damage, capability of vessel protection, realization of fine separation, minimal blood loss, easy operation under endoscope, avoidance or reduction of bile leakages and pleural effusions, and applicability for liver cirrhosis. Among the existing methods, single-orifice water jet technology has certain advantages; however, its defects such as low speed and difficult operation under endoscope also make it difficult for most surgeons to accept; in addition, according to failure analyses on existing water jet products, there are problems such as easy blockage and easy leakage, which also limits the promotion of these products.

At least one embodiment of the present disclosure provides a medical water jet scalpel including a water jet nozzle and a water jet tube. The water jet nozzle includes an inlet, an outlet and an inner lumen configured to provide a space for circulation of medical liquid. The inlet of the water jet tube is configured for an inflow of the medical liquid. The water jet nozzle includes a plurality of orifices, the plurality of orifices are communicated with the outlet of the water jet tube, and the plurality of orifices are configured to eject the medical liquid which passes through the outlet of the water jet tube.

At least one embodiment of the present disclosure also correspondingly provides a medical system including the medical water jet scalpel described above.

The above-described medical water jet scalpel provided by the embodiments of the present disclosure can realize multi-orifice cutting. Compared with the single-orifice cutting in the prior art, the soft tissues to be cut (e.g., substantial tissues) are difficult to escape from an impact area of multi-orifice jets, so that the cutting of the soft tissues and the separation of the soft tissues from the lumen-like elastic tissues can be completed in a relatively fixed manner, the performance of the water jet scalpel for selective tissue separation is enhanced, the surgery efficiency of the medical water jet scalpel for cutting (e.g., water jet liver resection) is improved, the surgery operation is simplified, and the problem that the water jet scalpel cannot work in case of orifice blockage can also be solved. Embodiments of the present disclosure and examples thereof are described in details below with reference to the accompanying drawings.

It should be noted that for convenience of description herein, in some embodiments of the present disclosure, an axial direction of the water jet tube is denoted as a left and right direction in the drawings, the side away from a liquid inflow end of the inner lumen is denoted as a left side, the side closer to the liquid inflow end is denoted as a right side, the inlet of the water jet tube is located at a right end of the inner lumen, and the outlet of the water jet tube is located at a left end of the inner lumen; the direction perpendicular to the left and right direction that is involved in some embodiments of the present disclosure can be denoted as an up and down direction in the drawings. It should be noted that all the orientations involved in the embodiments of the present disclosure represent the orientations in the illustrations of the drawings, without affecting the orientations in actual application, which is not limited in the present disclosure.

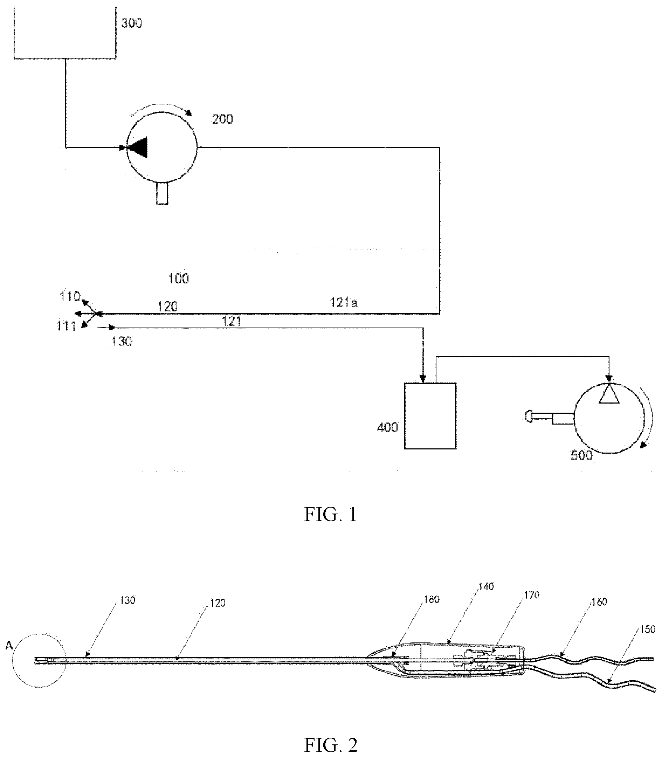

Fig. 1 is a schematic diagram of constituted components of a medical system provided by some embodiments of the present disclosure. Fig. 2 is a schematic structural diagram of a medical water jet scalpel provided by some embodiments of the present disclosure.

As shown in Fig. 1, the medical system includes a medical water jet scalpel 100, a pressure pump 200, and a liquid supply unit 300. The liquid supply unit 300 is configured to supply medical liquid. The pressure pump 200 is configured to apply a pressure to the medical liquid.

For example, in the embodiment of Fig. 1, the medical water jet scalpel 100 includes a water jet nozzle 110 and a water jet tube 120. The water jet nozzle 110 includes a plurality of orifices 111, i.e., the medical water jet scalpel 100 of the embodiment of the present disclosure may also be referred to as a medical multi-orifice water jet scalpel. The water jet tube 120 includes an inlet, an outlet and an inner lumen 121. The inner lumen 121 of the water jet tube 120 is configured to provide a space for circulation of the medical liquid. The medical liquid is driven to flow into the water jet tube 120 through the inlet 121a under a pressure applied by the pressure pump 200, and then flows through the inner lumen 121. Since the outlet (i.e., the left end in the figure) of the water jet tube 120 is communicated with a plurality of orifices 111, the medical liquid passes through the outlet of the water jet tube 120 and is ejected from the plurality of orifices 111.

For example, in some embodiments of the present disclosure, the liquid supply unit 300 includes a physiological saline bag, and the medical liquid includes physiological saline. Of course, this is merely illustrative and is not intended to be a limitation of the present disclosure, other suitable liquid supply units are also possible, e.g., a liquid reservoir, which may be configured depending on actual situations and will not be described in details herein.

For example, in the embodiment of Fig. 1, the medical system further includes a wastewater reservoir 400 and a negative pressure aspirator 500. As shown in Fig. 1 and Fig. 2, in some embodiments of the present disclosure, the medical water jet scalpel 100 further includes an outer aspiration tube 130 sleeved outside the water jet tube 120, and a gap space between an outer sidewall of the water jet tube 120 and an inner sidewall of the outer aspiration tube 130 serves as an aspiration channel. The wastewater reservoir 400 is communicated with the outer aspiration tube 130. The negative pressure aspirator 500 is connected to the wastewater reservoir 400 to provide a negative pressure capable of performing a suction function.

For example, in some embodiments of the present disclosure, an axial direction of the outer aspiration tube 130 is parallel to an axial direction of the water jet tube 120, for example, the outer aspiration tube 130 and the water jet tube 120 may be arranged coaxially; for example, in some other embodiments, the water jet tube 120 is sleeved outside the outer aspiration tube 130; it's also possible that the water jet tube 120 and the outer aspiration tube 130 are not arranged coaxially, for example, they may be arranged in parallel, which is configured depending on actual situations and will not be limited in the present disclosure.

For example, in the embodiment of Figs. 1 and 2, the medical water jet scalpel 100 further includes a handle 140 to which a right side of the outer aspiration tube 130 is fixedly connected. For example, the medical water jet scalpel 100 further includes an aspiration connecting tube 150 which is communicated with a right end of the outer aspiration tube 130; and a wastewater reservoir 400 is communicated with the outer aspiration tube 130 through the aspiration connecting tube 150 to collect waste liquid or tissue debris, etc. For example, the medical water jet scalpel 100 may further include an aspiration adapter 180, through which the communication between the aspiration connecting tube 150 and the outer aspiration tube 130 can be realized.

As shown in Fig. 2, the medical water jet scalpel 100 further includes an ejection connecting tube 160 which is communicated with an end of the water jet tube 120 away from the water jet nozzle 110. For example, the medical water jet scalpel 100 may further include an ejection adapter 170, through which the communication between the water jet tube 120 and the ejection connecting tube 160 can be realized.

Hereinafter, an operation method provided by some embodiments of the present disclosure with respect to the above-described medical system will be explained. The operation method includes the following steps: the liquid supply unit 300 supplies a working medium (i.e., medical liquid) to the pressure pump 200; the pressure pump 200 pressurizes the medical liquid to be at a working pressure under the drive of a program (control unit) in a host computer and of an electric motor; the medical liquid flows into the handle 140 through the ejection connecting tube 160, and reaches the water jet nozzle 110 after passing through the ejection adapter 170 and the water jet tube 120, and then is ejected, in the form of high-speed jets, from the plurality of orifices 111 in an end surface of the water jet nozzle 110. These jets are utilized to realize selective separation of soft tissues (e.g., liver parenchyma) from elastic tissues (e.g., blood vessels and bile ducts) in the body, for example, these jets are utilized to realize selective separation of liver parenchyma from lumen-like tissues.

For example, the pressure pump 200 is a high-pressure pump with a working pressure of 1 MPa to 10 MPa, although it's merely exemplary and is not a limitation of the present disclosure. In some embodiments of the present disclosure, the pressure pump 200 is connected to a host computer which includes a control unit and a power unit for controlling and driving the pressure pump 200, e.g., for adjusting a rotational speed. In some other embodiments, the pressure pump 200 itself has a power unit, or the pressure pump 200 is integrated with a control unit and a power unit. For example, the medical liquid is physiological saline; the medical water jet scalpel 100 is a disposable surgical instrument operatable by a single hand of a surgeon; and the selective separation of soft tissues from elastic tissues can be highly-efficiently performed by water jets generated from the plurality of orifices of the medical water jet scalpel 100.

Optionally, the operation method further includes: after the separation of the liver parenchyma from the lumen-like tissues is completed by using the jets, waste liquid, tissue debris and the like as generated will enter the outer aspiration tube 130 from the side close to the water jet nozzle 110, then flow through the aspiration channel and the aspiration adapter 180 to reach the aspiration connecting tube 150, and finally enter the wastewater reservoir 400, so that the ejected water and the tissues separated from the organs can be discharged out of the body through the tube lines. For example, the negative pressure aspirator 500 may provide a negative pressure of -0.02 MPa to -0.08 MPa in order to achieve a suction function.

For example, in some embodiments of the present disclosure, the water jet tube has an inner diameter of about 1.5 mm to 5 mm and an outer diameter of about 2 mm to 6 mm. For example, the orifice may have a diameter of about 0.05 mm to 0.15 mm. For example, in some embodiments of the present disclosure, the jet of the medical liquid ejected from the plurality of orifices 111 has a flow rate of 50 m/s to 100 m/s.

At present, there are only medical single-orifice water jet scalpels in the market, and conceptions or cases of a medical multi-orifice water jet scalpel for medical cutting separation are not yet available.

The medical multi-orifice water jet scalpel provided by the present disclosure at least solves the technical problems in the field of medical technology that the existing technology cannot realize effective separation by effectively cutting or removing soft tissues while completely preserving elastic tissues, and also archives many unexpected technical effects.

The inventors of the present disclosure have found that the medical single-orifice water jet scalpel in the prior art involves the following technical problems.
(1) In the case where the medical single-orifice water jet scalpel in the prior art is used for bombarding biological tissues with jets, since biological tissues having softness or elasticity are liable to move under bombardment, they may be shifted to the outside of the jet region under the action of the jets and hence easily escape from the range of the jet region. Since the cutting and separation effects of the medical single-orifice water jet scalpel are only concentrated in the jet region, if the soft tissues (e.g., liver parenchyma tissues) and the elastic tissues (e.g., lumen-like tissues) in the jet region are shifted under the action of the jets, the effects of selective tissue separation and the surgical accuracy achieved by the medical single-orifice water jet scalpel will be significantly affected, resulting in adverse surgical effects, for example, more liver parenchyma tissue residues accompanied therewith.
(2) For the medical single-orifice water jet scalpel in the prior art, in order to address the problem that soft biological tissues and elastic biological tissues are liable to escape, the diameter of the water flow is generally increased by increasing the ejected pressure of the jet or increasing the diameter of the single orifice of the medical single-orifice water jet scalpel so as to break the soft biological tissues as much as possible, thereby improving the efficiency of separating soft biological tissues from elastic biological tissues. However, this will lead to more ruptures and/or damages of elastic biological tissues (e.g., ruptures of blood vessels and damages of bile ducts), and conversely cause more adverse surgical consequences such as blood loss and edema of liver resection margin.
(3) Moreover, the medical single-orifice water jet scalpel in the prior art is operated in single-line mode. In order to complete the cutting and separation within an ideal exposed range of biological tissues, a medical single-orifice water jet scalpel movable at high frequency is required. This results in poor operation efficiency and greater difficulty in surgical operation.

The present disclosure has been made in response to the technical problems found by the inventors, and creates a medical multi-orifice water jet scalpel which can achieve numerous non-obvious technical effects listed as below, specifically.
(1) First of all, when the medical multi-orifice water jet scalpel provided by the present disclosure is utilized for cutting substantial tissues, selective separation of biological tissues can be realized efficiently and accurately. For example, the liver parenchyma tissue in the jet region can be accurately and highly efficiently cut off or removed, and meanwhile the blood vessels, bile ducts and the like distributed in the jet region are preserved from being damaged; for example, the elastic tissues such as blood vessels, bile ducts and the like, through which the jet travels, are prevented from being cut off. The completion of these two actions at the same time is of great significance for a liver resection surgery.

For example, in some embodiments, the medical multi-orifice water jet scalpel enables a selective separation of soft tissues from elastic tissues in a cutting zone having a certain width and length, for example, allowing the preserved blood vessels to have a certain exposed width (e.g., 3 mm to 5 mm), so as to facilitate the surgeon to carry out the next operation, i.e., precise coagulation and occlusion of blood vessels, after completing the exposure of the blood vessels (removing part of the liver parenchyma). Thus, the use of a medical multi-orifice water jet scalpel makes it possible for a cutting operation within a cutting zone having a certain width and length. In the case where the water jet nozzle of the medical multi-orifice water jet scalpel provided by the present disclosure moves slowly in a longitudinal direction while carrying out a rapid scanning movement in a transverse direction to form a reciprocating Z-shaped path, the cutting zone is completely covered and the blood vessels in the cutting zone (for example, the length is about 30 mm and the width is about 5 mm) are fully exposed, so that the liver parenchyma at the position where the jet passes through is cut off or removed, and lumen-like tissues such as blood vessels, bile ducts and the like associated with the liver parenchyma are preserved, thereby effectively realizing a selective separation. As such, the medical multi-orifice water jet scalpel of the present disclosure exhibits significant advantages in terms of the effects of selective tissue separation (e.g., protection of blood vessels, cutting depth, removal of substantial tissues) and the separation efficiency, as compared to a single-orifice water jet scalpel using the same parameter settings (e.g., the pressure of the jet, the diameter of the orifice, and the same human tissues).

Moreover, since the medical multi-orifice water jet scalpel of the present disclosure provides multiple streams of jets and the distance between jet regions corresponding to the multiple streams of jets is very small (e.g., only 0.4 mm to 4.0 mm), the soft or elastic biological tissue positioned between the multiple jet regions is limited by the multiple jet regions and thus cannot be freely shifted. For a medical multi-orifice water jet scalpel, during the movement of a cluster of jet regions of the water jet nozzle, the cutting of the soft tissues and the separation of the soft tissues from the lumen-like elastic tissues can be completed in a relatively fixed manner. The clustering effect generated in this way improves the actual working efficiency and working performance of the medical water jet scalpel. In other words, if biological tissues in a certain local area are hit by a jet from one of the orifices of the medical multi-orifice water jet scalpel, the biological tissues in this local area have a tendency of moving to adjacent positions where they may be hit by jets from other orifices in the medical multi-orifice water jet scalpel or have a tendency of moving in an opposite direction due to the jets of the other orifices, which achieves a fixed effect; that is, the actions of the plurality of orifices are complemented with each other so that the biological tissues have no way of escaping but can only be cut off or separated from the lumen-like elastic tissues during the process in which multiple streams of jets are ejected at the same time. Therefore, the multi-orifice water jet scalpel provided by the present disclosure not only increases the number of the orifices, but also achieves the above-mentioned technical effects that the technical skilled in the art cannot expect to obtain from the prior art and that the single-orifice water jet scalpel cannot realize, i.e., carrying out a selective separation of target biological tissues efficiently and accurately.

(2) Secondly, since the effects of selective separation achieved by the medical multi-orifice water jet scalpel of the present disclosure are greatly improved, the ejection pressure of the jets can be reduced in the surgical process. Thus, lumen-like tissues (e.g., small blood vessels and small bile ducts) can be protected and preserved better while satisfying efficient cutting and removal of substantial tissues. The preservation of lumen-like tissues (e.g., blood vessels and bile ducts) can reduce intraoperative bleeding and bile leakage, reduce postoperative complications and alleviate an edema caused by them, as well as shorten the length of hospital stay and time for postoperative recovery of patients after surgery.

(3) Thirdly, the medical multi-orifice water jet scalpel provided by the present disclosure is operated in a multi-line mode, so that when a selective separation is carried out in the cutting zone, the medical multi-orifice water jet scalpel only needs to be moved at a low frequency, which can achieve higher operation efficiency and also greatly reduce the difficulty in surgery operation.

Additionally, industrial jet machines fail to provide any technical enlightenment related to the medical multi-orifice water jet scalpel of the present disclosure, and the technical solutions of the medical multi-orifice water jet scalpel of the present disclosure are not easily conceived of based on the industrial jet machines.

First of all, high-pressure water jets are widely applied in the industry, but an industrial jet machine is mainly used for cleaning purposes in terms of functions and effects thereof. For example, it is easy to conceive of using multi-orifice jets for high-pressure cleaning, but the objective thereof is only to increase the water volume or expand the ejection area or improve the cleaning efficiency, i.e., there is no technical enlightenment of realizing selective separation. For example, industrial jets are carried out at high pressure and larger ejection area, and involve diversity in terms of ejected fluids, which makes it impossible to achieve the technical purpose of effectively cutting or removing soft tissues while completely preserving elastic tissues as much as possible, as required in the present disclosure.

Secondly, although industrial high-pressure water jets may occasionally be used for industrial high-pressure cutting, the cutting is merely carried out in the mode of single-orifice jet and there is no case for the mode of multi-orifice jet, because the industrial high-pressure cutting pursues for a cutting accuracy and a cutting efficiency but multi-orifice jet, if used for cutting, has no way of improving the cutting efficiency or the cutting accuracy; on the contrary, the multi-orifice jet will lead to a degradation of cutting accuracy, and the diversion of a single stream of jet into multiple streams under a constant pressure will cause a reduction in cutting efficiency. Further, tools for single-orifice jet cutting used in the industry cannot be directly applied to the medical multi-orifice water jet scalpel of the present disclosure, in view of materials, process difficulties, and costs.

Furthermore, the objects to be cut by the medical multi-orifice water jet scalpel of the present disclosure are not rigid bodies but very soft and elastic human tissues such as substantial tissues and lumen-like tissues (e.g., blood vessels, bile ducts). As comparison, the objects to be treated in the industrial field are rigid bodies (for example, metal plates) with extremely high hardness, so there is no technical problem that the objects to be treated are soft and are easy to move and escape from impingement of jets to cause adverse consequences. As a result, there is no technical demand for multi-orifice water jet in the industrial field, and naturally it wouldn't have occurred to conceive of applying the multi-orifice water jet into industrial cutting. Moreover, since the objects to be treated in the industrial field are rigid bodies (such as metal plates) with extremely high hardness, there is no need for a corresponding ideal exposure state and no need for a corresponding cutting zone. For example, all the cutting paths for the objects to be treated in the industrial field are linear (in straight lines and curves), resulting that the corresponding technical effects of the medical multi-orifice water jet scalpel of the present disclosure cannot be predicted and realized.

The medical multi-orifice water jet scalpel of the present disclosure and the industrial jet machines are confronted with essentially different technical problems, and belong to two quite different technical fields, with fundamentally different technical objectives to be achieved. The objective of the industrial jet machine is to achieve accurate, thorough and highly efficient cutting, while the objective of the medical multi-orifice water jet scalpel provided by the present disclosure is to realize a selective separation by effectively cutting or removing soft tissues while completely preserving elastic tissues as much as possible. The inventors of the present disclosure have also found that, exactly for the essentially different technical fields, technical problems to be addressed, and technical objectives to be achieved, the medical multi-orifice water jet scalpel provided by the present disclosure is distinguished from the industrial jet machine in the prior art in at least one or more of the following: (a) the objects to be cut by the industrial jet machine include rigid bodies such as metal, ceramic, glass, composite material, rock, and the like, while the objects to be cut by the medical multi-orifice water jet scalpel of the present disclosure are soft objects to be cut, for example, biological tissues such as parenchyma, mucosal layers; (b) the industrial jet machine generally employs water mixed with abrasive, e.g., carborundum and quartz sand, as the working medium, while the medical multi-orifice water jet scalpel of the present disclosure employs a medical liquid such as physiological saline; (c) the jet of the industrial jet machine has a flow rate of 800 m/s to 1000 m/s, while the water jet of the medical multi-orifice water jet scalpel provided by the present disclosure has a flow rate of 50 m/s to 100 m/s, i.e., there are differences of orders of magnitude therebetween; (d) the diameter of the orifice of the industrial jet machine is 0.2 mm to 1.0 mm, while the diameter of the orifice of the medical multi-orifice water jet scalpel of the present disclosure is 0.08 mm to 0.12 mm, i.e., there are differences of orders of magnitude therebetween; (e) the cutting width (Kerf) of the industrial jet machine is 0.22 mm to 1.1 mm and is about 10% larger than the diameter of the orifice thereof, while the cutting width (Kerf) realized by scanning of the medical multi-orifice water jet scalpel of the present disclosure is 3 mm to 5 mm and is about 30 to 50 times of the diameter of the orifice thereof, i.e., there are differences of orders of magnitude therebetween; (f) the working pressure of the industrial jet machine includes a low pressure of 30 Mpa to 50 Mpa, a high pressure of more than 100 MPa, and an ultra-high pressure of more than 200 MPa, while the working pressure of the medical multi-orifice water jet scalpel of the present disclosure is 1 Mpa to 10 Mpa, i.e., there are differences of orders of magnitude therebetween.

It should be noted that in the present disclosure, liver parenchyma and lumen-like tissues in a liver cancer surgery are mainly described as operation objects by way of example, but the medical multi-orifice water jet scalpel provided by the present disclosure is not limited to be only applied to liver cancer surgery; instead, it can also be applied to surgeries such as benign tumor resection, organ transplantation, and the like, and the operation objects of the medical multi-orifice water jet scalpel not only include liver parenchyma and lumen-like tissues corresponding to liver, but can also be applied to other biological tissues having similar requirements and characteristics, for example, substantial issues and lumen-like tissues (e.g., bile duct, ureter, bronchus, etc.) corresponding to other human organs (e.g., lung, kidney, spleen, etc.), which may be determined according to the actual situations and will not be limited to the embodiments of the present disclosure.

It should also be noted that in some embodiments, some of (no less than two of) the plurality of orifices (e.g., the number is greater than or equal to 2) of the water jet nozzle of the present disclosure may be utilized to eject the medical liquid so as to selectively separate the biological tissues, or all of the plurality of orifices may be utilized to eject the medical liquid so as to selectively separate the biological tissues, which is not limited in the present disclosure and may be determined depending on actual situations. For example, the medical multi-orifice water jet scalpel according to the following description allows for a surgical instrument to continue to operate even if some of the orifices are blocked, and the related contents will not be described here.

For example, in some embodiments of the present disclosure, a material of the water jet nozzle 110 includes one or more of the following: metal and gemstone. For example, the water jet nozzle 110 may be implemented as an artificial gemstone (e.g., ruby or sapphire), a metal sheet (e.g., a stainless-steel sheet), or the like.

For example, in some embodiments of the present disclosure, a material of the water jet tube 120 includes one or more of the following: metal and plastic. For example, the material of the water jet tube 120 includes stainless steel and/or polytetrafluoroethylene (PTFE).

For example, in some embodiments of the present disclosure, the plurality of orifices 111 are uniformly arrange. For example, the uniform arrangement of the plurality of orifices 111 may refer to that a pattern surrounded by the plurality of orifices 111 is a symmetrical pattern, and any orifice 111 and a corresponding adjacent orifice 111 have the same center distance. Of course, this is merely illustrative and is not a limitation of the present disclosure.

For example, in some embodiments of the present disclosure, a center distance of two adjacent orifices of the plurality of orifices 111 is 0.4 mm to 4.0 mm.

For example, an inner diameter of the outer aspiration tube 130 is 3 mm to 7.6 mm, and an outer diameter of the outer aspiration tube 130 is 4 mm to 8 mm. For example, a material of the outer aspiration tube 130 includes stainless steel and/or polysulfone plastic (PSU).

In some embodiments of the present disclosure, based on the medical multi-orifice water jet scalpel, multiple streams of water jets that are ejected in parallel may be concentrated within a small area (e. g., about 20 mm²) to provide a clustering effect, which brings in significant improvement in the degree of completion and the degree of penetration of selective separation, thereby increasing the cutting depth per unit time and improving the efficiency of selective separation.

For example, the ejection connecting tube 160 has an outer diameter of about 4 mm, an inner diameter of about 2 mm, and a length of about 3 m; and a material of the ejection connecting tube 160 may include PTFE.

For example, the aspiration connecting tube 150 has an outer diameter of about 8 mm, an inner diameter of about 6 mm, and a length of about 3 m; and a material of the aspiration connecting tube 150 may include polyvinyl chloride (PVC).

For example, a material of the ejection adapter 170 may include stainless steel and/or polyether-ether-ketone resin (PEEK).

For example, a material of the aspiration adapter 180 may include one or more of the following: acrylonitrile butadiene styrene copolymers (ABS), polycarbonate (PC), silica gel and rubber.

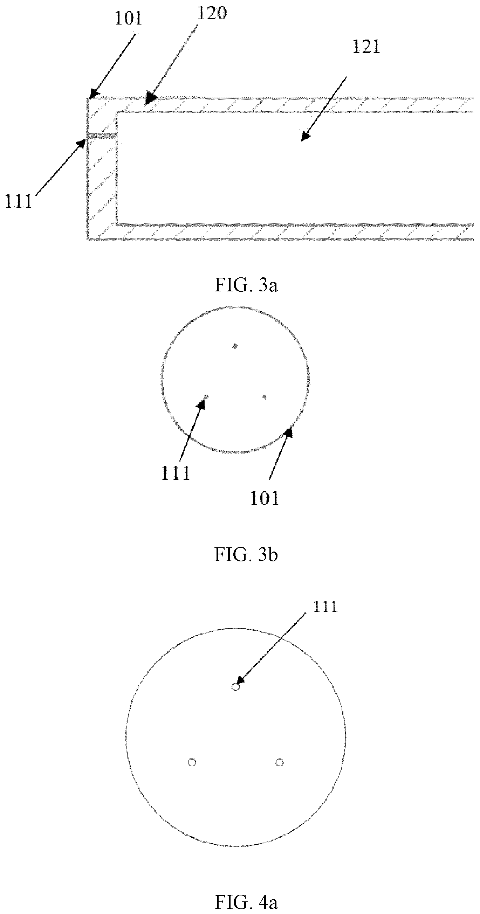

Fig. 3a is a schematic diagram of a first type of structure of a water jet nozzle and a water jet tube provided by some embodiments of the present disclosure, and Fig. 3b is a left side view of the first type of structure as shown in Fig. 3a.

As shown in Figs. 3a and 3b, a left end (which may also be referred to as a head end) of the water jet tube 120 is a blind end 101, with at least a part of the blind end 101 serving as the water jet nozzle 110.

For example, in the embodiment of Figs. 3a and 3b, a material of the water jet tube 120 includes stainless steel and/or PTFE. The water jet tube 120 has an outer diameter of about 3 mm and an inner diameter of about 2 mm. The blind end 101 has a wall thickness of about 1 mm. For example, the plurality of orifices 111 are obtained by directly machining micropores with a diameter of 0.1mm in a left end surface of the blind end 101. For example, the number of the plurality of orifices 111 in the left end surface of the blind end 101 is three and these three orifices 111 are uniformly arranged, that is, any two adjacent orifices 111 have the same center distance, and center points of the three orifices 111 are symmetrically arranged around a center point of an end surface of the blind end 101.

Fig. 4a is a schematic diagram illustrating a uniform arrangement of three orifices provided by some embodiments of the present disclosure.

As shown in Fig. 4a, the number of the plurality of orifices 111 of the medical water jet scalpel 100 is three, and these three orifices 111 are uniformly arranged. For example, in the embodiment of Fig. 4a, the three orifices 111 are sequentially connected to form an equilateral triangle, and any two adjacent orifices 111 have the same center distance which is in the range of 0.4 mm to 4.0 mm.

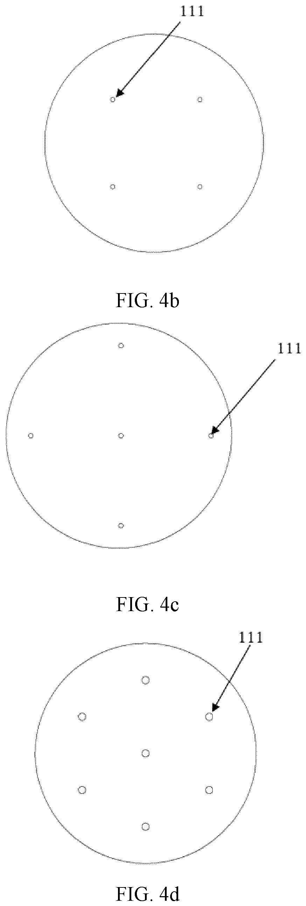

Fig. 4b is a schematic diagram illustrating a uniform arrangement of four orifices provided by some embodiments of the present disclosure.

As shown in Fig. 4b, the number of the plurality of orifices 111 of the medical water jet scalpel 100 is four, and these four orifices 111 are uniformly arranged. For example, in the embodiment of Fig. 4b, the four orifices 111 are sequentially connected to form a square, and any two adjacent orifices 111 have the same center distance which is in the range of 0.4 mm to 4.0 mm.

Fig. 4c is a schematic diagram illustrating a uniform arrangement of five orifices provided by some embodiments of the present disclosure.

As shown in Fig. 4c, the number of the plurality of orifices 111 of the medical water jet scalpel 100 is five, and these five orifices 111 are uniformly arranged. For example, in the embodiment of Fig. 4c, one of the five orifices 111 is located at a central position and the other four orifices 111 are uniformly distributed around a periphery of the central orifice 111, wherein the four orifices 111 at the periphery are sequentially connected to form a square, any two adjacent orifices 111 of the four orifices 111 at the periphery have the same center distance which is in the range of 0.4 mm to 4.0 mm, and the central orifice 111 and any one of the orifices 111 at the periphery also have the same center distance.

Fig. 4d is a schematic diagram illustrating a uniform arrangement of seven orifices provided by some embodiments of the present disclosure.

As shown in Fig. 4d, the number of the plurality of orifices 111 of the medical water jet scalpel 100 is seven, and these seven orifices 111 are uniformly arranged. For example, in the embodiment of Fig. 4d, one of the seven orifices 111 is located at a central position and the other six orifices 111 are uniformly distributed around a periphery of the central orifice 111; the six orifices 111 at the periphery are sequentially connected to form a regular hexagon, any two adjacent orifices of the six orifices 111 at the periphery have the same center distance which is in the range of 0.4 mm to 4.0 mm, and the central orifice 111 and any one of the orifices 111 at the periphery also have the same center distance.

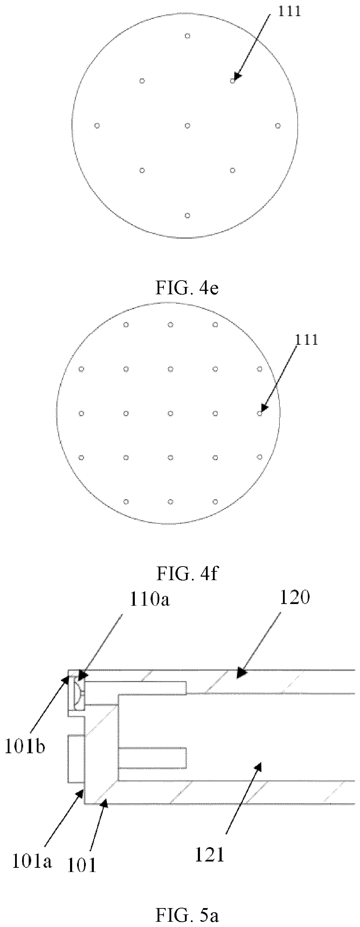

Fig. 4e is a schematic diagram illustrating a uniform arrangement of nine orifices provided by some embodiments of the present disclosure.

As shown in Fig. 4e, the number of the plurality of orifices 111 of the medical water jet scalpel 100 is nine, and these nine orifices 111 are uniformly arranged. For example, in the embodiment of Fig. 4e, one of the nine orifices 111 is located at a central position and the other eight orifices 111 are uniformly distributed at a periphery around the central orifice 111, wherein the eight orifices 111 at the periphery are sequentially connected to form a square, and any two adjacent orifices of the eight orifices 111 at the periphery have the same center distance; in the square formed by sequentially connecting the eight orifices 111 at the periphery, both diagonals of the square pass through the central orifice 111, the center distance between any two adjacent ones of the eight orifices 111 at the periphery is in the range of 0.4 mm to 4.0 mm, and the center distance between the central orifice 111 and any one of the orifices 111 at the periphery is also in the range of 0.4 mm to 4.0 mm.

Fig. 4f is a schematic diagram of a uniform arrangement of twenty-one orifices provided by some embodiments of the present disclosure.

As shown in Fig. 4f, the number of the plurality of orifices 111 of the medical water jet scalpel 100 is twenty-one, and the twenty-one orifices 111 are uniformly arranged. For example, as illustrated in Fig. 4f, one of the twenty-one orifices 111 is located at a central position, and the other twenty orifices 111 are uniformly distributed around the central orifice 111 at a periphery thereof, layer by layer, along the up and down direction and the left and right direction. For example, the twenty-one orifices 111 are arranged in five rows and five columns, each of the three rows in the middle includes five orifices 111, and each of the upper and lower rows includes three orifices, and any two adjacent orifices 111 in each row or column have the same center distance which is in the range of 0.4 mm to 4.0 mm.

It should be noted that neither the number of the plurality of orifices 111 included in the medical water jet scalpel 100 nor the specific layout of the plurality of orifices is limited in the present disclosure, which will not be described in detail here.

It should also be noted that according to some embodiments of the present disclosure, it is not easy to realize the design of a water jet nozzle having a plurality of orifices in the medical water jet scalpel 100, because there are technical difficulties as follows: (a) a diameter of an outer tube (e.g., the outer aspiration tube 130) of the medical water jet scalpel 100 is limited to be no more than 5 mm, because the outer tube at the left side (closer to the outlet) of the medical water jet scalpel 100 needs to be protruded into the patient's body during the surgery after punctured with a hole by a piercer, and the piercer capable of puncturing generally has corresponding specifications including, for example, but not limited to, a diameter of 5 mm to 10 mm, depending on actual situations, whereby the diameter of the outer tube of the medical water jet scalpel 100 should not exceed a reasonable range of the corresponding specifications, for example, a range of 5 mm to 5.4 mm corresponding to the specifications of 5 mm in diameter; (b) it is also necessary to arrange an aspiration channel having a corresponding cross-sectional area (for example, not less than 5 mm² corresponding to the specifications of 5 mm in diameter) in the outer tube of the medical water jet scalpel 100; (3) the orifice has a relatively smaller diameter, for example, about 0.08 mm to 0.12 mm, which is difficult to process and realize; (4) in view of the needs of jets, a surface roughness of the end surface of the orifice and a surface roughness of the cylindrical surface of the water jet tube are required to be as small as possible, for example, it is generally required that the surface roughness is no more than Ra 0.4 µm; (5) the high-pressure water jet tube and parts/fittings of the orifices still need to be positioned, fixed and sealed in designs; (6) the cost for a handle of a disposable medical water jet scalpel needs to be reduced as far as possible. Therefore, due to the above-described technical difficulties, it is not easy for a person skilled in the art to conceive of the conceptions and technical solutions of the medical multi-orifice water jet scalpel in the foregoing corresponding embodiments.

For example, in some embodiments of the present disclosure, the water jet nozzle 110 includes a multi-orifice sub-nozzle provided with a plurality of orifices 111 formed therein. For example, in some other embodiments of the present disclosure, the water jet nozzle 110 includes a plurality of single-orifice sub-nozzles, and each of the plurality of orifices 111 is arranged on a corresponding one of the plurality of single-orifice sub-nozzles. It should be noted that whether the water jet nozzle 110 employs a multi-orifice sub-nozzle or a plurality of single-orifice sub-nozzles is not limited in the embodiments of the present disclosure, and may be designed according to different structural forms of the water jet nozzle, for example, which will be illustrated below.

It should be noted that the orifices involved herein may be regular orifices or irregular orifices, which is not limited in the present disclosure; furthermore, the description of the sizes of the orifices involved herein is not intended to limit the specific shapes of the orifices of the embodiments of the present disclosure, although the orifices are generally regarded as circular orifices and the sizes of the orifices are represented by their diameters or radii, which are not the key points required to be illustrated in the present disclosure and are not described in detail herein.

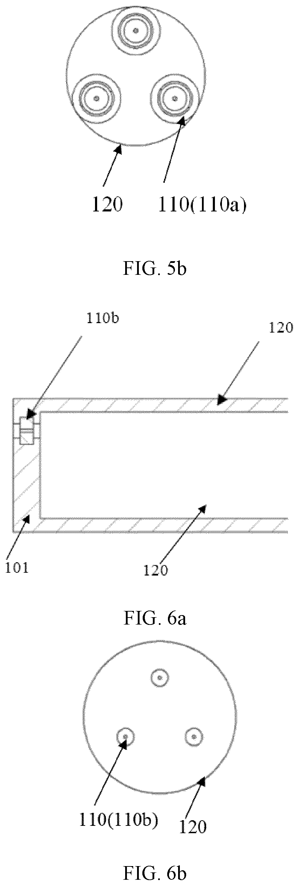

For example, in some embodiments of the present disclosure, the left end of the water jet tube 120 is a blind end, and the water jet nozzle 110 is arranged at the blind end of the water jet tube 120, for example, in an embedded manner, as shown in Figs. 5a and 5b, and Figs. 6a and 6b, for example.

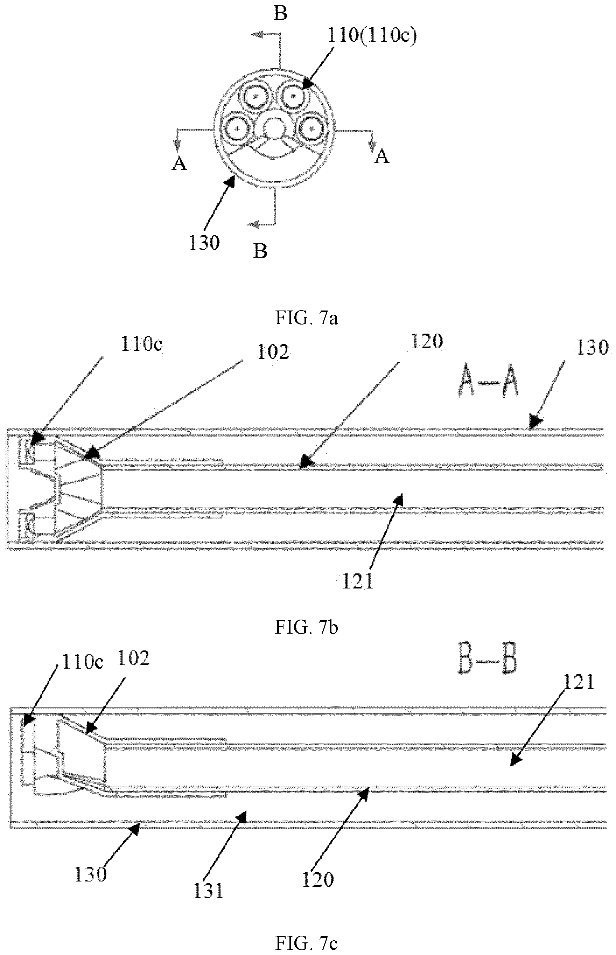

For example, in some other embodiments of the present disclosure, the water jet tube 120 is a sleeved tube opened at both of the left and right ends, the water jet nozzle 110 is fixed at the left end of the sleeved tube, and the water jet nozzle 110 is connected with the left end of the sleeved tube in a sealed manner, directly or indirectly, as shown in Figs. 7a and 7b, 8a and 8b, 9a and 9b, 10a and 10b, and 11a and 11b, for example.

Fig. 5a is a schematic diagram of a second type of structure of a water jet nozzle and a water jet tube as provided by some embodiments of the present disclosure, and Fig. 5b is a left side view of the second type of structure as shown in Fig. 5a.

As shown in Figs. 5a and 5b, the blind end 101 of the water jet tube 120 is formed with a stepped orifice for the water jet nozzle 110 to be embedded. For example, the blind end 101 of the water jet tube 120 includes one blind end surface 101a and a plurality of stepped orifices 101b. The stepped orifice 101b includes a first subspace protruded from the blind end surface and a second subspace recessed into the blind end surface, and each of the plurality of single-orifice sub-nozzles 110a included in the water jet nozzle 110 is correspondingly embedded into the first subspace of one of the plurality of stepped orifices 101b. For example, in the embodiment of Fig. 5b, if the number of the plurality of single-orifice sub-nozzles 110a included in the water jet nozzle 110 is three, the number of the stepped orifices 101b is also three, which, of course, is merely exemplary and is not a limitation of the present disclosure.

For example, in the embodiment of Figs. 5a and 5b, a material of the water jet tube 120 includes stainless steel, and the water jet tube 120 has an outer diameter of about 3 mm and an inner diameter of about 2.4 mm. For example, the single-orifice sub-nozzle 110a in the embodiment of Fig. 5a is implemented as a single-orifice gemstone, having an outer diameter of about 1 mm and a thickness of about 0.3 mm.

For example, in the embodiment of Fig. 5a and Fig. 5b, a single-orifice sub-nozzle 110a is placed into a stepped orifice 101b at the left end of the water jet tube 120, and a protruded portion of the stepped orifice of the water jet tube 120 is inwardly crimped by a stamping process so that the single-orifice sub-nozzle 110a is fixed in a sealed manner.

Fig. 6a is a schematic diagram of a third type of structure of a water jet nozzle and a water jet tube as provide by some embodiments of the present disclosure, and Fig. 6b is a left side view of the third type of structure as shown in Fig. 6a.

As shown in Figs. 6a and 6b, the left end of the water jet tube 120 is a blind end 101. For example, the water jet nozzle 110 includes a plurality of single-orifice sub-nozzles 110b embedded in the blind end 101 at the left end of the water jet tube 120. For another example, the water jet nozzle 110 includes a multi-orifice sub-nozzle 110b embedded in the blind end 101 at the left end of the water jet tube 120.

For example, in the embodiment of Figs. 6a and 6b, a material of the water jet tube 120 include PPS plastic, the water jet tube 120 has an outer diameter of about 3 mm and an inner diameter of about 2 mm. For example, the single-orifice sub-nozzle 110b in the embodiment of Figs. 6a and 6b has an outer diameter of about 1 mm and a thickness of about 0.3mm.

For example, in the embodiment of Figs. 6a and 6b, a plurality of single-orifice sub-nozzles 110b will be fixed in a sealed manner during an injection molding process of the water jet tube 120 by placing the plurality of single-orifice sub-nozzles 110b as embedded parts into an injection mold.

Fig. 7a is a schematic diagram illustrating a fourth type of structure of a water jet nozzle and a water jet tube provide by some embodiments of the present disclosure, and Fig. 7b and 7c are schematic diagrams illustrating sections A-A and B-B, respectively, of the fourth type of structure as shown in Fig. 7a.

As shown in Figs. 7a-7c, the left end of the medical water jet scalpel 100 further includes a nested joint 102 which is fixedly connected with the water jet tube 120 in a sealed manner, and the water jet nozzle 110 is connected with the water jet tube 120 through the nested joint 102; a counterbore is arranged at a left end of the nested joint 102, the water jet nozzle 110 is arranged in the counterbore of the nested joint 102, and a sidewall of the counterbore wraps the water jet nozzle 110. The orifices 111, the nested joint 102, and the water jet tube 120 are sequentially communicated along multiple axes of the orifices 111 parallel to the water jet tube 120 so that the medical liquid flowing into the water jet tube 120 through the right end thereof is smoothly ejected from the plurality of orifices 111.

For example, in the embodiment of Figs. 7a-7c, the outer aspiration tube 130 is sleeved outside the water jet tube 120, and a gap space 131 between an outer sidewall of the water jet tube 120 and an inner sidewall of the outer aspiration tube 130 serves as an aspiration channel.

For example, in the embodiment of Fig. 7a-7c, the water jet nozzle 110 includes a plurality of single-orifice sub-nozzles 110c, and each of the plurality of orifices 111 is arranged in a corresponding one of the plurality of single-orifice sub-nozzles 110c.

For example, in the embodiment of Figs. 7a-7c, the outer aspiration tube 130 includes stainless steel; and the outer aspiration tube 130 has an outer diameter of about 5 mm, an inner diameter of about 4.6 mm, and a length of about 400 mm. The water jet tube 120 includes stainless steel; and the water jet tube 120 has an outer diameter of about 2.2 mm and an inner diameter of about 2 mm. For example, in the embodiment of Fig. 7a, the nested joint 102 is made of metal, a material of the nested joint 102 includes stainless steel, and the nested joint 102 has a special-shaped lumen for connecting the water jet nozzle 110 and the water jet tube 120. For example, in the embodiment of Fig. 7a, the single-orifice sub-nozzle 110c is a single-orifice gemstone having an outer diameter of about 1 mm and a thickness of about 0.3 mm.

For example, in the embodiment of Figs. 7a-7c, the water jet nozzle 110 and the water jet tube 120 are connected with each other, and a circumferential welding process is further performed at a joint therebetween for fixing and sealing; then a single-orifice sub-nozzle 110c is put into the counterbore at the left end of the nested joint 102, and a part deep in the sidewall of the counterbore of the nested joint 102 is crimped by a stamping process so as to wrap the single-orifice sub-nozzle 110c.

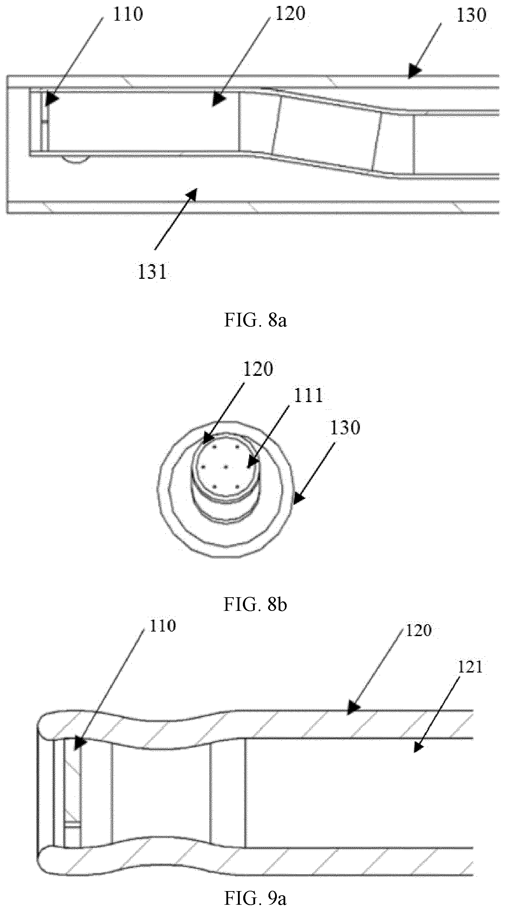

Fig. 8a is a schematic diagram of a fifth type of structure of a water jet nozzle and a water jet tube as provided by some embodiments of the present disclosure, and Fig. 8b is a left side view of the fifth type of structure as shown in Fig. 8a.

As shown in Figs. 8a and 8b, the left end of the water jet tube 120 is in interference fit with the water jet nozzle 110. For example, in the embodiment of Figs. 8a and 8b, the outer aspiration tube 130 is sleeved outside the water jet tube 120, and a gap space 131 between an outer sidewall of the water jet tube 120 and an inner sidewall of the outer aspiration tube 130 serves as an aspiration channel.

For example, in the embodiment of Figs. 8a and 8b, the water jet nozzle 110 is a multi-orifice sub-nozzle 110 which is tightly held in place and sealed by heating the water jet tube 120 to expand its inner bore to have a diameter greater than the outer diameter of the water jet nozzle 110, placing the water jet nozzle 110 into the inner bore of the water jet tube 120, and cooling the water jet tube 120 to be contracted.

For example, in the embodiment of Figs. 8a and 8b, a material of the outer aspiration tube 130 may include stainless steel and/or PSU; the outer aspiration tube 130 has an outer diameter of about 5 mm and an inner diameter of about 4.6 mm. For example, in the embodiment of Figs. 8a and 8b, the water jet tube 120 includes stainless steel, and the water jet tube 120 has an outer diameter of about 3 mm and an inner diameter of about 2.6 mm. For example, in the embodiment of Figs. 8a and 8b, the multi-orifice sub-nozzle 110 is a monolithic multi-orifice gemstone having an outer diameter of about 2.6 mm and a thickness of about 0.3 mm.

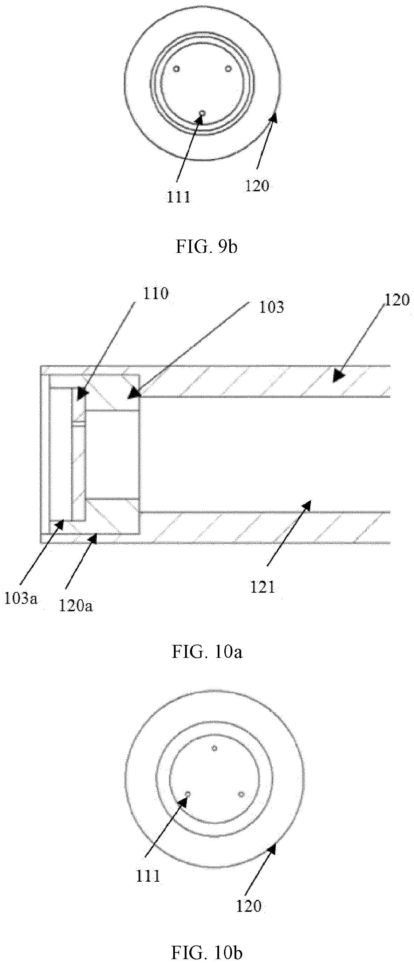

Fig. 9a is a schematic diagram of a sixth type of structure of a water jet nozzle and a water jet tube as provided by some embodiments of the present disclosure, and Fig. 9b is a left side view of the sixth type of structure as shown in Fig. 9a.

As shown in Figs. 9a and 9b, the water jet nozzle 110 is fixed at an inner side of the left end of the water jet tube 120 in a sealed manner. For example, in the embodiment of Fig. 9a and Fig. 9b, the water jet tube 120 is a sleeved tube of PTFE (polytetrafluoroethylene), and the water jet nozzle 110 is a multi-orifice sub-nozzle 110. For example, the fixing and sealing of the multi-orifice sub-nozzle 110 is achieved by fitting the multi-orifice sub-nozzle 110 into a left head end inside the water jet tube 120, and then deforming the left head end of the water jet tube 120 in a thermal contraction manner by heating and pressing from the outside of the water jet tube 120.

For example, in the embodiment of Figs. 9a and 9b, the water jet tube 120 has an outer diameter of about 3 mm and an inner diameter of about 2 mm. For example, in Figs. 9a and 9b, the multi-orifice sub-nozzle 110 is a single, multi-orifice gemstone having an outer diameter of about 2 mm and a thickness of about 0.3 mm.

Fig. 10a is a schematic diagram of a seventh type of structure of a water jet nozzle and a water jet tube as provided by some embodiments of the present disclosure, and Fig. 10b is a left side view of the seventh type of structure as shown in Fig. 10a.

As shown in Figs. 10a and 10b, the left end of the medical water jet scalpel 100 further includes a protective cover 103 which is opened at both of left and right ends; the left end of the water jet tube 120 is provided with a first counterbore 120a, and the protective cover 103 is embedded into the first counterbore 120a of the water jet tube 120; the left end of the protective cover 103 is provided with a second counterbore 103a, and the water jet nozzle 110 is embedded into the second counterbore 103a of the protective cover 103. The orifices 111, the protective cover 103, and the water jet tube 120 are sequentially communicated along multiple axes of the orifices 111 parallel to the water jet tube 120, so that the medical liquid flowing into the water jet tube 120 through the right end thereof can be smoothly ejected from the plurality of orifices 111.

For example, in the embodiment of Figs. 10a and 10b, a material of the water jet tube 120 includes stainless steel, and the water jet tube 120 has an outer diameter of about 3 mm and an inner diameter of about 2.4 mm; the left end of the water jet tube 120 has a step for inwardly crimping. For example, in the embodiment of Figs. 10a and 10b, a material of the protective cover 103 may include copper and/or PTFE; the protective cover 103 has an outer diameter of about 2.6 mm and an inner diameter of about 2 mm; and the left end of the protective cover 103 is provided with a step for holding the water jet nozzle 110. For example, in the embodiment of Figs. 10a and 10b, the water jet nozzle 110 is a multi-orifice sub-nozzle 110, which is a monolithic multi-orifice gemstone; the water jet nozzle 110 has an outer diameter of about 2.2 mm and a thickness of about 0.3 mm, and a spacing between adjacent orifices is about 1 mm.

For example, in the embodiment of Fig. 10a and Fig. 10b, the multi-orifice sub-nozzle 110 is fitted into the second counterbore 103a of the protective cover 103; the protective cover 103 is fitted into the first counterbore 120a of the water jet tube 120; an outer wall of the left end of the water jet tube 120 is crimped inwardly, by stamping, for fixing the protective cover 103 and the multi-orifice sub-nozzle 110; and the outer wall of the left end of the protective cover 103 is deformed under pressure so as to wrap a left side surface of the multi-orifice sub-nozzle 110. Considering the brittleness, the small thickness and the friability of the material of the multi-orifice sub-nozzle 110, the protective cover 103 having a certain elasticity is provided as a buffer and a sealer.

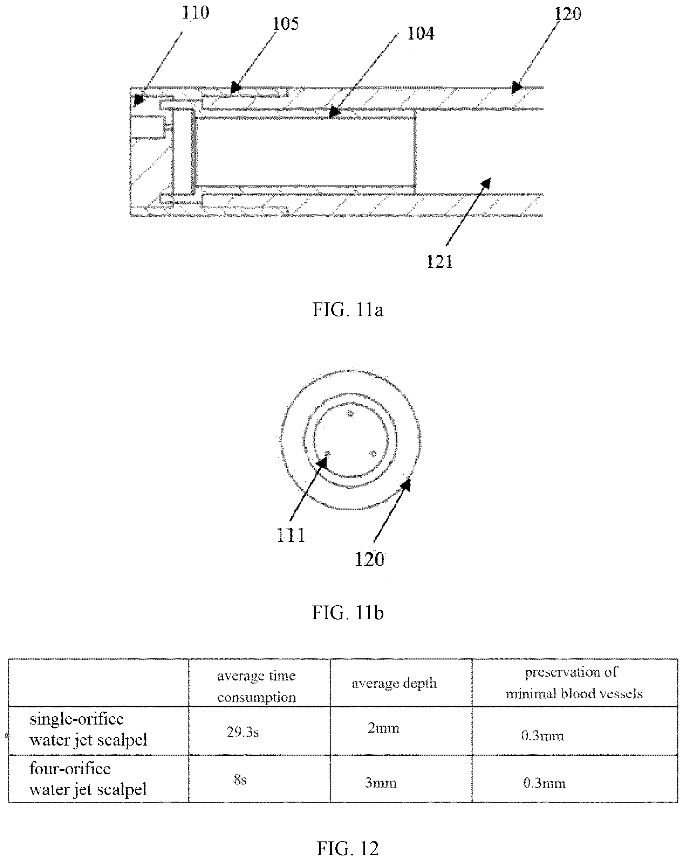

Fig. 11a is a schematic diagram of an eighth type of structure of a water jet nozzle and a water jet tube as provided by some embodiments of the present disclosure, and Fig. 11b is a left side view of the eighth type of structure as shown in Fig. 11a.

As shown in Figs. 11a and 11b, the left end of the medical water jet scalpel 100 further includes a support sleeve 104; the outer sidewall of the left end of the water jet tube 120 is configured as a step structure, the support sleeve 104 is matched with the step structure of the water jet tube 120 in shape, and the support sleeve 104 is protruded into the inner lumen of the water jet tube 120; the water jet nozzle 110 is fixedly connected at a left end of the support sleeve 104; and a part of a space outside an integral structure formed by connecting the step structure of the water jet tube 120, the support sleeve 104 and the water jet nozzle 110 is used as an injection molding region 105.

For example, in the embodiment of Figs. 11a and 11b, by connecting the extruded water jet tube 120, the stamped support sleeve 104 and the injecting-molded water jet nozzle 110 together and by tightly compressing the same in an axial direction, a secondary injection molding process is performed in the injection molding region 105 (i.e., a secondary injection molding region), that is, the secondary injection molding process is performed on an outer side surface of a circumference of the left head end; and after an injection molding material flows into this space, it is fused with the water jet tube 120 and the water jet nozzle 110 of the same material so as to achieve fixing and sealing.

For example, in the embodiment of Fig. 11a and 11b, a material of the water jet tube 120 includes PTFE, and the water jet tube 120 has an outer diameter of about 3 mm and an inner diameter of about 2 mm. For example, a material of the support sleeve 104 includes stainless steel, and the support sleeve 104 is sized to cooperate with the water jet tube 120 and the water jet nozzle 110, respectively, for purpose of positioning, and for preventing the material from infiltration into the water jet tube 120 during the secondary injection molding process. For example, in the embodiment of Figs. 11a and 11b, the water jet nozzle 110 is a multi-orifice sub-nozzle 110, a material of the water jet nozzle 110 includes PTFE, and the water jet nozzle 110 has an outer diameter of about 2.6 mm and a thickness of about 1 mm; the water jet nozzle 110 includes three orifices which are uniformly distributed and have a spacing of about 1 mm. For example, a material of the secondary injection molding region includes PTFE.

Fig. 12 is a schematic table illustrating comparison results of separation efficiency and capability of a medical single-orifice water jet scalpel vs a medical multi-orifice water jet scalpel, as provided by some embodiments of the present disclosure.

As shown in Fig. 12, the performance of selective separation performed by the medical water jet scalpel is mainly reflected by the following three aspects: the average time consumption of the separation operation, the depth of the separated region per unit time and the preservation of lumen-like tissues (such as small blood vessels/bile ducts). For example, a medical four-orifice water jet scalpel was compared with the existing medical single-orifice water jet scalpel, and the experimental data are particularly shown in the table of Fig. 12. As can be seen from the data in the table, compared with the medical single-orifice water jet scalpel, the medical multi-orifice water jet scalpel provided by the embodiments of the present disclosure can remarkably improve the working efficiency (i.e., the average time consumption) and the working performance (i.e., the average depth of the separated region) without sacrificing the advantage of preserving the small vessels. Moreover, the number of the orifices, the spacing of the orifices and the layout mode of the orifices in the multi-orifice nozzle all provide more spaces for performance optimization and application range expansion of the multi-orifice water jet scalpel.

At least one embodiment of the present disclosure employs a medical multi-orifice water jet scalpel, which can solve the technical problems of low efficiency, poor effect, and difficult operation during the selective separation process of biological tissues in the prior art (as described above), as compared with the existing medical single-orifice water jet scalpel. Therefore, the medical multi-orifice water jet scalpel of the present disclosure makes itself stand out from huge number of liver resection instruments to provide a surgical instrument with high performance, high efficiency and convenient usage for the surgeons.

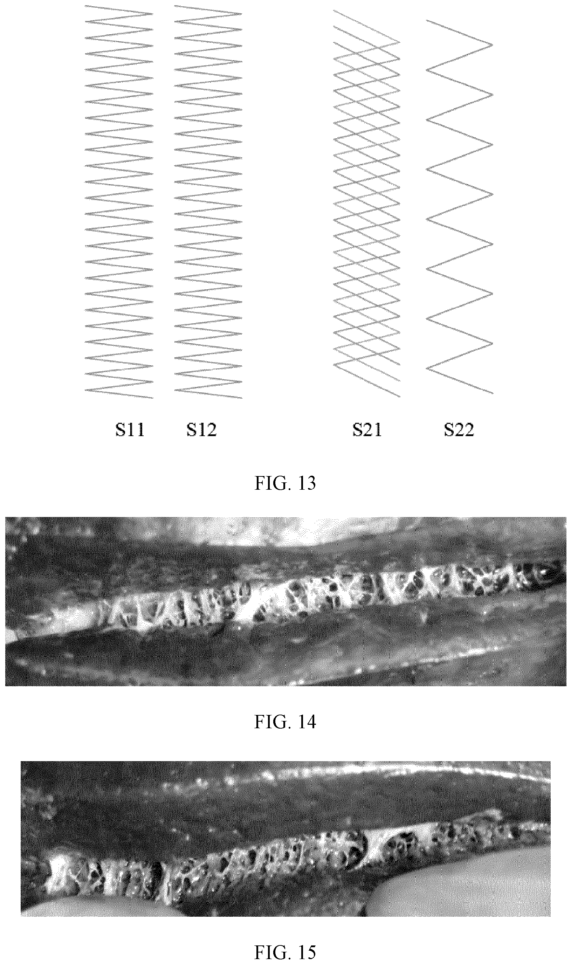

Fig. 13 is a schematic diagram illustrating an operation path of a medical single-orifice water jet scalpel and an operation path of a medical multi-orifice water jet scalpel provided by some embodiments of the present disclosure.

As shown in Fig. 13, the path S 1 1 represents an operation path of orifice of a medical single-orifice water jet scalpel, the path S 12 represents an operation path of a handle of a medical single-orifice water jet scalpel, the path S21 represents an operation path of orifices of a medical multi-orifice water jet scalpel (e.g., a medical three-orifice water jet scalpel), and the path S22 represents an operation path of a handle of a medical multi-orifice water jet scalpel (e.g., a medical three-orifice water jet scalpel), in which the up and down direction in Fig. 13 represents a length direction of a cutting zone, and the left and right direction in Fig. 13 represents a width direction of the cutting zone.

For example, as described above, a medical multi-orifice water jet scalpel is required to simultaneously accomplish, for example, the cutting or removal of liver parenchyma as well as preservation and exposure of luminal-like tissues in a cutting zone having certain width and length, for example, the width of the cutting zone may be up to 30 to 50 times of the diameter of the orifice. For example, the jet of the medical water jet scalpel is a slender water column with a small diameter (e.g., 0.1mm), and the medical water jet scalpel can only produce the effects of cutting and crushing on a path through which the jet passes; as a result, in order to expose lumen-like tissues of a certain part (e.g., blood vessels and bile ducts of a certain part of the liver), a surgeon needs to make a path of the jet completely cover this area, which can be achieved by the surgeon by way of scanning as shown in Fig. 13, thereby forming a reciprocating Z-shaped path.

For example, where it is necessary to expose blood vessels in an area with a lateral width of 5 mm and a longitudinal length of 30 mm, in order to realize selective separation of full coverage, an impact point of the jet may be swung to the left and right while moving downwards slowly to achieve a coverage scan of the entire area. For the medical single-orifice water jet scalpel, the path of the impact point of the jet is exactly the same as the path of the orifice and the path of the handle. As comparison, for the medical multi-orifice water jet scalpel, multiple streams of jets are ejected simultaneously, and each transverse movement of the handle is equivalent to the simultaneous transverse scanning movements of the multiple streams of jets. Therefore, only a small amount of lateral movement of the handle of the medical multi-orifice water jet scalpel is required to complete the coverage scan of the corresponding area.

With reference to the embodiment of Fig. 13, the medical single-orifice water jet scalpel is operated in a single-line mode, and the medical multi-orifice water jet scalpel is operated in a multi-line mode. For example, the time required for a liver resection performed by a medical single-orifice water jet scalpel is about 60 minutes, and the time required for a liver resection performed by a medical three-orifice water jet scalpel is about 20 minutes. In theory, the number of the transverse movements of the handle of a medical multi-orifice water jet scalpel (e.g., a medical n-orifice water jet scalpel) is basically only 1/n of the number of the transverse movements of the handle of a medical single-orifice water jet scalpel; accordingly, the operation time of the medical multi-orifice water jet scalpel is shortened to nearly 1/n of that of the medical single-orifice water jet scalpel, which also means that the multi-orifice jet can improve a coverage area of the jet cutting action per unit time, improve the working efficiency of the medical water jet scalpel, and thus improve the surgical efficiency. Moreover, the working efficiency and the working performance can be further improved, which is benefited from the clustering effect produced by the multi-orifice jet described above.

In terms of the operation of the handle, the existing medical single-orifice water jet scalpel is transversely oscillated at a high frequency, while the medical multi-orifice water jet scalpel provided by the embodiments of the present disclosure only needs to transversely oscillate at a low frequency, so that the difficulty of surgery operation can be greatly reduced; the complex operation is one of the pain points for surgeons to use the medical single-orifice water jet scalpel, particularly in an environment of endoscopic surgery. Therefore, the difference between the single-orifice water jet scalpel and the multi-orifice water jet scalpel in clinical application is more obvious.

It should be noted that the medical multi-orifice water jet scalpel according to at least one embodiment of the present disclosure can also solve the problem that the entire instrument may be ineffective due to orifice blockage, which is easy to occur with the medical single-orifice water jet scalpel.

For example, the failure rate of the existing medical single-orifice water jet scalpel due to orifice blockage is about 20%, and the orifice blockage in the medical single-orifice water jet scalpel can directly cause the surgical equipment to be completely unable to work and repair and seriously affect the surgical procedure. In the case where a medical multi-orifice water jet scalpel is adopted, for example, if a medical four-orifice water jet scalpel is adopted, the failure rate of the medical four-orifice water jet scalpel that is unable to work due to complete orifice blockage can be reduced to be less than 0.16%; for another example, if a medical seven-orifice water jet scalpel is adopted, the failure rate of the medical seven-orifice water jet scalpel that is unable to work due to complete orifice blockage can be reduced to be less than 0.00128%, far less than the failure rate of the medical single-orifice water jet scalpel. Therefore, the medical multi-orifice water jet scalpel can become a trustable instrument for surgeons.

Fig. 14 is a schematic diagram illustrating processing effects of a medical multi-orifice water jet scalpel provided by some embodiments of the present disclosure; and Fig. 15 is a schematic diagram illustrating processing effects of a medical single-orifice water jet scalpel provided by some embodiments of the present disclosure.

For example, in some embodiments, the substantial tissue residue in Fig. 14 is much less than the substantial tissue residue in Fig. 15, and it can be seen from Fig. 14 that almost all the substantial tissues are cut off and removed; the depth of the separated region in Fig. 14 is greater than that in Fig. 15, and the effect of preserving the lumen-like tissues in Fig. 14 is superior to that in Fig. 15. Therefore, the effect of selective separation achieved by the medical multi-orifice water jet scalpel is far superior to that achieved by the medical single-orifice water jet scalpel.

In summary, the embodiments of the present disclosure propose a design for the medical multi-orifice water jet scalpel according to clinical requirements, which can improve the surgical efficiency and surgical effect of selective separation performed by the water jet scalpel on the basis of reserving the technical advantages of the existing medical single-orifice water jet scalpel, simplify the surgical operation, and also solve the problem that the water jet scalpel cannot work in case of orifice blockage, thereby providing more satisfied technical solutions for corresponding surgical treatments.

It should be noted that in the foregoing embodiments of the present disclosure, the technical solutions of the medical water jet scalpel are described by dividing the medical water jet scalpel into elements or articles for performing corresponding functions. However, it is clear to the person skilled in the art that the functions performed by the elements or articles may be carried out under the above-mentioned divisions or in other division manners, which is not intended to limit the scope of protection of the present disclosure. Moreover, the meanings and functions of the elements or articles in the above embodiments of the present disclosure are not limited by their names and cannot be explained in an idealized or highly formal sense.

The following points need to be clarified:
(1) The drawings of the embodiments of the present disclosure only refer to the structures to which the embodiments of the present disclosure relate, and other structures may refer to general designs.
(2) In case of no conflict, the embodiments of the present disclosure and the features in the embodiments may be combined with each other to obtain new embodiment(s).

The above description is only about specific implementations of the present disclosure, but the scope of protection of the present disclosure is not limited thereto. The scope of protection of the present disclosure should be subject to the scope of protection of the appended claims.

## Claims

1. A medical water jet scalpel, comprising:
a water jet tube, comprising an inlet, an outlet, and an inner lumen configured to provide space for circulation of medical liquid, wherein the inlet is configured for an inflow of the medical liquid; and
a water jet nozzle, comprising a plurality of orifices, wherein the plurality of orifices are communicated with the outlet of the water jet tube, and the plurality of orifices are configured to eject the medical liquid passing through the outlet.

2. The medical water jet scalpel according to claim 1, wherein
the water jet nozzle comprises a multi-orifice sub-nozzle provided with the plurality of orifices.

3. The medical water jet scalpel according to claim 1 or 2, wherein
the water jet nozzle comprises a plurality of single-orifice sub-nozzles, and at least part of the plurality of orifices are respectively formed in the plurality of single-orifice sub-nozzles.

4. The medical water jet scalpel according to any one of claims 1-3, wherein
the outlet of the water jet tube is a blind end, and at least part of the blind end serves as the water jet nozzle.

5. The medical water jet scalpel according to claim 2 or 3, wherein
the outlet of the water jet tube is a blind end, and the water jet nozzle is arranged at the blind end of the water jet tube.

6. The medical water jet scalpel according to claim 2 or 3, wherein
the water jet tube is a sleeved tube opened at both ends, and
the water jet nozzle is fixed at the outlet of the water jet tube, and the water jet nozzle is connected with the outlet of the water jet tube in a sealed manner, directly or indirectly.

7. The medical water jet scalpel according to claim 6, further comprising an outer aspiration tube, wherein
the outer aspiration tube is sleeved outside the water jet tube, and
a gap space between an outer sidewall of the water jet tube and an inner sidewall of the outer aspiration tube serves as an aspiration channel.

8. The medical water jet scalpel according to claim 7, wherein
an inner diameter of the outer aspiration tube is 3 mm to 7.6 mm, and an outer diameter of the outer aspiration tube is 4 mm to 8 mm;
an inner diameter of the water jet tube is 1.5 mm to 5 mm, and an outer diameter of the water jet tube is 2 mm to 6 mm; and
a diameter of the orifice is 0.05 mm to 0.15 mm.

9. The medical water jet scalpel according to any one of claims 1-8, wherein
the plurality of orifices are uniformly arranged.

10. The medical water jet scalpel according to any one of claims 1-9, wherein
a distance between centers of two adjacent orifices among the plurality of orifice is 0.4 mm to 4.0 mm.

11. The medical water jet scalpel according to any one of claims 1-10, wherein
a flow rate of a jet of the medical liquid ejected from the plurality of orifices is 50 m/s to 100 m/s.

12. The medical water jet scalpel according to any one of claims 1-11, wherein
the water jet nozzle comprises one or more of the following materials: metal, gemstone and plastic; and
the water jet tube comprises one or more of the following materials: metal and plastic.

13. The medical water jet scalpel according to any one of claims 6-8, further comprising a nested joint, wherein
the nested joint is fixedly connected with the water jet tube in a sealed manner, and the water jet nozzle is connected with the water jet tube through the nested joint;
an output end of the nested joint is provided with a counterbore, the water jet nozzle is arranged in the counterbore of the nested joint, and a sidewall of the counterbore wraps the water jet nozzle, and
the plurality of orifices, the nested j oint and the water jet tube are sequentially communicated along multiple axes of the plurality of orifices parallel to the water jet tube.

14. The medical water jet scalpel according to claim 6, wherein
the water jet nozzle is fixed at an inner side of the outlet of the water jet tube in a sealed manner.

15. The medical water jet scalpel according to claim 6, further comprising a protective cover which is opened at both ends, wherein
the outlet of the water jet tube is provided with a first counterbore, and the protective cover is embedded in the first counterbore of the water jet tube,
an output end of the protective cover is provided with a second counterbore, and the water jet nozzle is embedded in the second counterbore of the protective cover, and
the plurality of orifices, the protective cover and the water jet tube are sequentially communicated along multiple axes of the plurality of orifices parallel to the water jet tube.

16. The medical water jet scalpel according to claim 3 or 5, wherein
the outlet of the water jet tube is a blind end, and the water jet nozzle is arranged at the blind end of the water jet tube;
the blind end of the water jet tube comprises one blind end surface and a plurality of stepped holes, wherein the stepped hole comprises a first subspace protruded from the blind end surface and a second subspace recessed into the blind end surface; and
each single-orifice sub-nozzle of the plurality of single-orifice sub-nozzles of the water jet nozzle is embedded in the first subspace of a corresponding stepped hole of the plurality of stepped holes.

17. The medical water jet scalpel according to claim 6, further comprising a support sleeve, wherein
the outlet of the water jet tube is configured as a step structure, the support sleeve is matched with the step structure of the water jet tube in shape and protrudes into the inner lumen of the water jet tube, and the water jet nozzle is fixedly connected with an output end of the support sleeve; and
a part of a space outside the integral structure constituted by connecting the step structure, the support sleeve and the water jet nozzle is used as an injection molding region.

18. The medical water jet scalpel according to claim 7 or 8, further comprising a handle, wherein
the side of the outer aspiration tube away from the water jet nozzle is fixedly connected with the handle.

19. The medical water jet scalpel according to claim 7 or 8 or 18, further comprising an aspiration connecting tube, wherein
the aspiration connecting tube is communicated with an end of the outer aspiration tube away from the water jet nozzle.

20. The medical water jet scalpel according to claim 7 or 8 or 18 or 19, further comprising an ejection connecting tube, wherein
the ejection connecting tube is communicated with an end of the water jet tube away from the water jet nozzle.

21. The medical water jet scalpel according to any one of claims 1-20, wherein
the plurality of orifices comprise at least two orifices configured to eject the medical liquid to selectively separate a target tissue.

22. A medical system, comprising:
the medical water jet scalpel according to any one of claims 1-21;
a liquid supply unit configured to provide the medical liquid; and
a pressure pump configured to apply a pressure to the medical liquid to input the medical liquid into the inner lumen of the water jet tube.

23. The medical system according to claim 22, further comprising a wastewater reservoir, wherein
the medical water jet scalpel also comprises an outer aspiration tube, wherein the outer aspiration tube is sleeved outside the water jet tube, and a gap space between an outer sidewall of the water jet tube and an inner sidewall of the outer aspiration tube is used as an aspiration channel, and
the wastewater reservoir is communicated with the outer aspiration tube.

24. The medical system according to claim 23, further comprising a negative pressure aspirator, wherein
the negative pressure aspirator is connected to the wastewater reservoir.
